# EUROPEAN PATENT SPECIFICATION

(11) **EP 1 842 845 B1**
(45) Date of publication and mention of the grant of the patent: **16.07.2014**
(21) Application number: 07007352.3
(22) Date of filing: 03.12.1998
(51) Int. Cl.: C07D 207/16, A61K 31/401, C07D 207/12, A61P 17/14, A61P 25/28

(54) **AZA-HETEROCYCLIC COMPOUNDS USED TO TREAT NEUROLOGICAL DISORDERS AND HAIR LOSS**
AZAHETEROZYKLISCHEDERIVATE ZUR BEHANDLUNG VON HAARAUSFALL UND NEUROLOGISCHER KRANKHEITEN
COMPOSÉS AZA-HETEROCYCLIQUES UTILISÉS POUR TRAITER LES TROUBLES NEUROLOGIQUES ET LA PERTE DES CHEVEUX

(30) Priority: 03.06.1998 US 87788 P; 18.09.1998 US 101077 P
(43) Date of publication of application: 10.10.2007
(62) Divisional of application: 98961866.5
(73) Proprietor: GliaMed, Inc., New York, NY 10032 (US)
(72) Inventor: Hamilton, Gregory S., Catonsville, MD 21228 (US); Norman, Mark H., Thousand Oaks, CA 91360 (US); Wu, Yong-Qian, Columbia, MD 21044 (US)
(74) Representative: Smaggasgale, Gillian Helen

(56) References cited:
- WO-A-92/00278
- WO-A-96/40633

## Description

### BACRGROUND OF THE INVENTION

### 1. Field of the Invention

This invention relates to novel carboxylic acid and carboxylic acid isosteres of N-heterocylic compounds, their preparation and use for treating neurological disorders including physically damaged nerves and neurodegenerative diseases, and for treating alopecia and promoting hair growth.

### 2. Description of the Prior Art

It has been found that picomolar concentrations of an immunosuppressant such as FK506 and rapamycin stimulate neurite out growth in PC12 cells and sensory nervous, namely dorsal root ganglion cells (DRGs). Lyons et al., Proc. of Natl. Acad. Sci., 1994 vol. 91, pp. 3191-3195.

In whole animal experiments, FK506 has been shown to stimulate nerve regeneration following facial nerve injury and results in functional recovery in animals with sciatic nerve lesions.

Several neurotrophic factors effecting specific neuronal populations in the central nervous system have been identified. For example, it has been hypothesized that Alzheimer's disease results from a decrease or loss of nerve growth factor (NGF). It has thus been proposed to treat Alzheimer's patients with exogenous nerve growth factor or other neurotrophic proteins such as brain derived nerve factor (BDNF), glial derived nerve factor, ciliary neurotrophic factor, and neurotropin-3 to increase the survival of degenerating neuronal populations.

Clinical application of these proteins in various neurological disease states is hampered by difficulties in the delivery and bioavailability of large proteins to nervous system targets. By contrast, immunosuppressant drugs with neurotrophic activity are relatively small and display excellent bioavailability and specificity. However, when administered chronically, immunosuppressants exhibit a number of potentially serious side effects including nephrotoxicity, such as impairment of glomerular filtration and irreversible interstitial fibrosis (Kopp et al., 1991, J. Am. Soc. Nephrol. 1:162); neurological deficits, such as involuntary tremors, or non-specific cerebral angina such as non-localized headaches (De Groen et al., 1987, N. Engl. J. Med. 317:861); and vascular hypertension with complications resulting therefrom (Kahan et al., 1989 N. Engl. J. Med. 321: 1725).

Accordingly, there is a need for small-molecule compounds which are useful for neurotrophic effects and for treating neurodegenerative disorders.

Hair loss occurs in a variety of situations. These situations include male pattern alopecia, alopecia senilis, alopecia areata, diseases accompanied by basic skin lesions or tumors, and systematic disorders such as nutritional disorders and internal secretion disorders. The mechanisms causing hair loss are very complicated, but in some instances can be attributed to aging, genetic disposition, the activation of male hormones, the loss of blood supply to hair follicles, and scalp abnormalities.

The immunosuppressant drugs FK506, rapamycin and cyclosporin are well known as potent T-cell specific immunosuppressants, and are effective against graft rejection after organ transplantation. It has been reported that topical, but not oral, application of FK506 (Yamamoto et al., J. Invest. Dermatol., 1994, 102, 160-164; Jiang et al., J. Invest. Dermatol. 1995, 104, 523-525) and cyclosporin (Iwabuchi et al., J. Dermatol. Sci. 1995, 9, 64-69) stimulates hair growth in a dose-dependent manner. One form of hair loss, alopecia areata, is known to be associated with autoimmune activities; hence, topically administered immunomodulatory compounds are expected to demonstrate efficacy for treating that type of hair loss. The hair growth stimulating effects of FK506 have been the subject of an international patent filing covering FK506 and structures related thereto for hair growth stimulation (Honbo et al., EP 0 423 714 A2). Honbo et al. discloses the use of relatively large tricyclic compounds, known for their immunosuppressive effects, as hair revitalizing agents.

The hair growth and revitalization effects of FK506 and related agents are disclosed in many U.S. patents (Goulet et al., U.S. Patent No. 5,258,389; Luly et al., U.S. Patent No. 5,457,111; Goulet et al., U.S. Patent No. 5,532,248; Goulet et al., U.S. Patent No. 5,189,042; and Ok et al., U.S. Patent No. 5,208,241; Rupprecht et al., U.S. Patent No. 5,284,840; Organ et al., U.S. Patent No. 5,284,877). These patents claim FK506 related compounds. Although they do not claim methods of hair revitalization, they disclose the known use of FK506 for affecting hair growth. Similar to FK506 (and the claimed variations in the Honbo et al. patent), the compounds claimed in these patents are relatively large. Further, the cited patents relate to immunomodulatory compounds for use in autoimmune related diseases, for which FK506's efficacy is well known.

Other U.S. patents disclose the use of cyclosporin and related compounds for hair revitalization (Hauer et al., U.S. Patent No. 5,342,625; Eberle, U.S. Patent No. 5,284,826; Hewitt et al., U.S. Patent No. 4,996,193). These patents also relate to compounds useful for treating autoimmune diseases and cite the known use of cyclosporin and related immunosuppressive compounds for hair growth.

However, immunosuppressive compounds by definition suppress the immune system and also exhibit other toxic side effects. Accordingly, there is a need for small molecule compounds which are useful as hair revitalizing compounds.

### SUMMARY OF THE INVENTION

The present invention relates to the surprising discovery that N-heterocyclic compounds containing a carboxylic acid or carboxylic acid isostere moiety may be useful for treating neurodegenerative disorders and for treating alopecia and related hair loss disorders. Accordingly, a novel class of compounds containing an acidic moiety or an isostere thereof attached to the 2-carbon of the N-heterocyclic ring is provided. These compounds stimulate neuronal regeneration and outgrowth and as such are useful for treating neurological disorders and neurodegenerative diseases. These compounds also promote hair growth and as such are useful for treating hair loss disorders. A preferred feature of the compounds of the present invention is that they do not exert any significant immunosuppressive activity and/or are non-immunosuppressive.

According to the present invention there is provided:
A pharmaceutical composition, comprising
   a) an effective amount of a compound according to formula 3, 5, 8, 21, or 137: 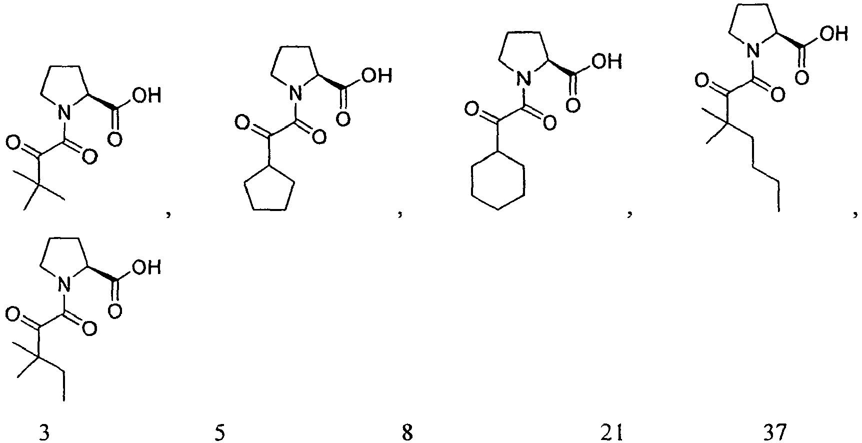 or a pharmaceutically acceptable salt thereof, and
   b) a pharmaceutically acceptable carrier.

For neurotrophic compositions an additional neurotrophic factor may also be administered or otherwise included in the composition.

The compounds of the present invention are useful in a method of promoting neuronal regeneration and growth in mammals.

The compounds are also useful in a method of treating a neurological disorder in an animal by stimulating growth of damaged peripheral nerves or to promote neuronal regeneration.

The compounds are also useful in a method of preventing neurodegeneration in an animal.

The compounds are also useful in a method of treating alopecia or promoting hair growth in an animal.

### BRIEF DESCRIPTION OF THE DRAWINGS

Figure 1 is a photograph of C57 Black 6 mice before being shaved for the hair regeneration experiment.
Figure 2 is a photograph of mice treated with a vehicle after six weeks. Figure 2 shows that less than 3% of the shaved area is covered with new hair growth when the vehicle (control) is administered.
Figure 3 is a bar graph illustrating relative hair growth on shaved mice treated with N-heterocyclic carboxylic acids or carboxylic acid isosteres at 1µmole per milliliter three times per week. Hair growth was evaluated after 14 days of treatment.

### DETAILED DESCRIPTION OF THE INVENTION

### Definitions

The term "pharmaceutically acceptable salt, ester, or solvate" refers to salt, ester, or solvates of the subject compounds which possess the desired pharmacological activity and which are neither biologically nor otherwise undesirable. The salt, ester, or solvates can be formed with inorganic or organic acids such as acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, gluconate, glycerophosphate, hemisulfate, heptanoate, hexanoate, hydrochloride hydrobromide, hydroiodide, 2-hydroxyethanesulfonate, lactate, maleate, methanesulfonate, naphthylate, 2-naphthalenesulfonate, nicotinate, oxalate, sulfate, thiocyanate, tosylate and undecanoate. Base salt, ester, or solvates include ammonium salts, alkali metal salts such as lithium, sodium and potassium salts, alkaline earth metal salts such as calcium and magnesium salts, salt with organic bases such as dicyclohexylamine salts, N-methyl-D-glucamine, and salts with amino acids such as arginine, lysine, and so forth. Also, the basic nitrogen-containing groups can be quarternized with such agents as: 1) lower alkyl halides, such as methyl, ethyl, propyl, and butyl chloride, bromides and iodides; 2) dialkyl sulfates like dimethyl, diethyl, dibutyl and diamyl sulfates; 3) long chain alkyls such as decyl, lauryl, myristyl and stearyl substituted with one or more halide such as chloride, bromide and iodide; and 4) aralkyl halides like benzyl and phenethyl bromide and others.

The compounds of this invention may possess at least one asymmetric center and thus can be produced as mixtures of stereoisomers or as individual enantiomers or diastereomers. The individual stereoisomers may be obtained by using an optically active starting material, by resolving a racemic or non-racemic mixture of an intermediate at some appropriate stage of the synthesis, or by resolution of the compound of formula (I). It is understood that the individual stereoisomers as well as mixtures (racemic and non-racemic) of stereoisomers are encompassed by the scope of the present invention. The S-stereoisomer is a most preferred embodiment of the invention.

"Stereoisomers" are isomers that differ only in the way the atoms are arranged in space.

"Isomers" are different compounds that have the same molecular formula and includes cyclic isomers such as (iso)indole and other isomeric forms of cyclic moieties.

"Enantiomers" are a pair of stereoisomers that are non-superimposable mirror images of each other.

"Diastereoisomers" are stereoisomers which are not mirror images of each other.

"Racemic mixture" means a mixture containing equal parts of individual enantiomers. "Non-racemic mixture" is a mixture containing unequal parts of individual enantiomers or stereoisomers.

The term "preventing neurodegeneration" as used herein includes the ability to inhibit or prevent neurodegeneration in patients newly diagnosed as having a neurodegenerative disease, or at risk of developing a new degenerative disease and for inhibiting or preventing further neurodegeneration in patients who are already suffering from or have symptoms of a neurodegenerative disease when the compounds are given concurrently.

The term "treatment" as used herein covers any treatment of a disease and/or condition in an animal, particularly a human, and includes:
(i) preventing a disease and/or condition from occurring in a subject which may be predisposed to the disease and/or condition but has not yet been diagnosed as having it;
(ii) inhibiting the disease and/or condition, i.e., arresting its development; or
(iii) relieving the disease and/or condition, i.e., causing regression of the disease and/or condition.

"Alopecia" refers to deficient hair growth and partial or complete loss of hair, including without limitation androgenic alopecia (male pattern baldness), toxic alopecia, alopecia senilis, alopecia areata, alopecia pelada and trichotillomania. Alopecia results when the pilar cycle is disturbed. The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae, and the hairs fall out. Alopecia has a number of etiologies, including genetic factors, aging, local and systemic diseases, febrile conditions, mental stresses, hormonal problems, and secondary effects of drugs.

"Pilar cycle" refers to the life cycle of hair follicles, and includes three phases:
(1) the anagen phase, the period of active hair growth which, insofar as scalp hair is concerned, lasts about three to five years;
(2) the catagen phase, the period when growth stops. and the follicle atrophies which, insofar as scalp hair is concerned, lasts about one to two weeks; and
(3) the telogen phase, the rest period when hair progressively separates and finally falls out which, insofar as scalp hair is concerned, lasts about three to four months.
Normally 80 to 90 percent of the follicles are in the anagen phase, less than 1 percent being in the catagen phase, and the rest being in the telogen phase. In the telogen phase, hair is uniform in diameter with a slightly bulbous, non-pigmented root. By contrast, in the anagen phase, hair has a large colored bulb at its root.

"Promoting hair growth" refers to maintaining, inducing, stimulating, accelerating, or revitalizing the germination of hair.

"Treating alopecia" refers to:
(i) preventing alopecia in an animal which may be predisposed to alopecia; and/or
(ii) inhibiting, retarding or reducing alopecia; and/or
(iii) promoting hair growth; and/or
(iv) prolonging the anagen phase of the hair cycle; and/or
(v) converting vellus hair to growth as terminal hair. Terminal hair is coarse, pigmented, long hair in which the bulb of the hair follicle is seated deep in the dermis. Vellus hair, on the other hand, is fine, thin, non-pigmented short hair in which the hair bulb is located superficially in the dermis. As alopecia progresses, the hairs change from the terminal to the vellus type.

The term "neurotrophic" as used herein includes without limitation the ability to stimulate neuronal regeneration or growth and/or the ability to prevent or treat neurodegeneration.

The term "non-immunosuppressive" refers to the inability of the compounds of the present invention to trigger an immune response when compared to a control such as FK506 ro cyclosporin A. Assays for determining immunosuppression are well known to those of ordinary skill in the art. Specific non-limiting examples of well known assays include PMA and OKT3 assays wherein mitogens are used to stimulate proliferation of human peripheral blood lymphocytes (PBC). Compounds added to such assay systems are evaluated for their ability to inhibit such proliferation.

### Compounds of the Invention

The present invention relates to the surprising discovery that carboxylic acid compounds are neurotrophic and are able to treat alopecia. Accordingly, a novel class of compounds are provided. A preferred feature of the compounds of the present invention is that they do not exert any significant immunosuppressive activity.

The compounds of this invention can be periodically administered to a patient undergoing treatment for neurological disorders or for other reasons in which it is desirable to stimulate neuronal regeneration and growth, such as in various peripheral neuropathic and neurological disorders relating to neurodegeneration. The compounds of this invention can also be administered to mammals other than humans for treatment of various mammalian neurological disorders.

The novel compounds of the present invention possess an excellent degree of neurotrophic activity. This activity is useful in the stimulation of damaged neurons, the promotion of neuronal regeneration, the prevention of neurodegeneration, and in the treatment of several neurological disorders known to be associated with neuronal degeneration and peripheral neuropathies. The neurological disorders that may be treated include but are not limited to: trigeminal neuralgia, glossopharyngeal neuralgia, Bell's Palsy, myasthenia gravis, muscular dystrophy, amyotrophic lateral sclerosis, progressive muscular atrophy, progressive bulbar inherited muscular atrophy, herniated, ruptured or prolapsed invertebrate disk syndromes, cervical spondylosis, plexus disorders, thoracic outlet destruction syndromes, peripheral neuropathic such as those caused by lead, dapsone, ticks, prophyria, or Gullain-Barré syndrome, Alzheimer's disease, and Parkinson's disease.

The above discussion relating to the utility and administration of the compounds of the present invention also applies to the pharmaceutical compositions of the present invention.

The term "pharmaceutically acceptable carrier" as used herein refers to any carrier, diluent, excipient, suspending agent, lubricating agent, adjuvant, vehicle, delivery system, emulsifier, disintegrant, absorbant, preservative, surfactant, colorant, flavorant, or sweetener.

For these purposes the compounds of the present invention may be administered orally, parenterally, by inhalation spray, topically, rectally, nasally, buccally, vaginally or via an implanted reservoir in dosage formulations containing conventional non-toxic pharmaceutically-acceptable carriers, adjuvants and vehicles. The term parenteral as used herein includes subcutaneous, intravenous, intramuscular, intraperitoneally, intrathecally, intraventricularly, intrasternal and intracranial injection or infusion techniques.

For oral administration, the compounds of the present invention may be provided in any suitable dosage form known in the art. For example, the compositions may be incorporated into tablets, powders, granules, beads, chewable lozenges, capsules, liquids, aqueous suspensions or solutions, or similar dosage forms, using conventional equipment and techniques known in the art. Tablet dosage forms are preferred. Tablets may contain carriers such as lactose and corn starch, and/or lubricating agents such as magnesium stearate. Capsules may contain diluents including lactose and dried corn starch. Aqueous suspensions may contain emulsifying and suspending agents combined with the active ingredient.

When preparing dosage form incorporating the compositions of the invention, the compounds may also be blended with conventional excipients such as binders, including gelatin, pregelatinized starch, and the like; lubricants, such as hydrogenated vegetable oil, stearic acid, and the like; diluents, such as lactose, mannose, and sucrose; disintegrants, such as carboxymethylcellulose and sodium starch glycolate; suspending agents, such as povidone, polyvinyl alcohol, and the like; absorbents, such as silicon dioxide; preservatives, such as methylparaben, propylparaben, and sodium benzoate; surfactants, such as sodium lauryl sulfate, polysorbate 80, and the like; colorants such as F.D.& C. dyes and lakes; flavorants; and sweeteners.

Compositions and the uses thereof of the invention also may utilize controlled release technology. Thus, for example, the inventive compounds may be incorporated into a hydrophobic polymer matrix for controlled release over a period of days. Such controlled release films are well known to the art. Particularly preferred are transdermal delivery systems. Other examples of polymers commonly employed for this purpose that may be used in the present invention include nondegradable ethylene-vinyl acetate copolymer and degradable lactic acid-glycolic acid copolymers which may be used externally or internally. Certain hydrogels such as poly(hydroxyethylmethacrylate) or poly(vinylalcohol) also may be useful, but for shorter release cycles then the other polymer releases systems, such as those mentioned above.

To be effective therapeutically as central nervous system targets, the compounds of the present invention should readily penetrate the blood-brain barrier when peripherally administered. Compounds which cannot penetrate the blood-brain barrier can be effectively administered by an intraventricular route or other appropriate delivery system suitable for administration to the brain.

The compounds of the present invention may be administered in the form of sterile injectable preparations, for example, as sterile injectable aqueous or oleaginous suspensions. These suspensions may be formulated according to techniques known in the art using suitable dispersing or wetting agents and suspending agents. The sterile injectable preparations may also be sterile injectable solutions or suspensions in non-toxic parenterally-acceptable diluents or solvents, for example, as solutions in 1,3-butanediol. Among the acceptable vehicles and solvents that may be employed are water, Ringer's solution and isotonic sodium chloride solution. In addition, sterile, fixed oils are conventionally employed as solvents or suspending mediums. For this purpose, any bland fixed oil may be employed including synthetic mono- or di-glycerides. Fatty acids such as oleic acid and its glyceride derivatives, including olive oil and castor oil, especially in their polyoxyethylated versions, are useful in the preparation of injectables. These oil solutions or suspensions may also contain long-chain alcohol diluents or dispersants.

The compounds of this invention may also be administered rectally in the form of suppositories. These compositions can be prepared by mixing the drug with a suitable non-irritating excipient which is solid at room temperature, but liquid at rectal temperature and, therefore, will melt in the rectum to release the drug. Such materials include cocoa butter, beeswax and polyethylene glycols.

The compounds of this invention may also be administered topically, especially when the conditions addressed for treatment involve areas or organs readily accessible by topical application, including neurological disorders of the eye, the skin, or the lower intestinal tract. Suitable topical formulations are readily prepared for each of these areas.

For topical application to the eye, or ophthalmic use, the compounds can be formulated as micronized suspensions in isotonic, pH adjusted sterile saline, or, preferably, as solutions in isotonic, pH adjusted sterile saline, either with or without a preservative such as benzylalkonium chloride. Alternatively for the ophthalmic uses the compounds may be formulated in an ointment such as petrolatum.

For topical application to the skin, the compounds can be formulated in a suitable ointment containing the compound suspended or dissolved in, for example, a mixture with one or more of the following: mineral oil, liquid petrolatum, white petrolatum, propylene glycol, polyoxyethylene polyoxypropylene compound, emulsifying wax and water. Alternatively, the compounds can be formulated in a suitable lotion or cream containing the active compound suspended or dissolved in, for example, a mixture of one or more of the following: mineral oil, sorbitan monostearate, polysorbate 60, cetyl esters wax, cetearyl alcohol, 2-octyldodecanol, benzyl alcohol and water.

Topical application for the lower intestinal tract an be effected in a rectal suppository formulation (see above) or in a suitable enema formulation.

Dosage levels on the order of about 0.1 mg to about 10,000 mg of the active ingredient compound are useful in the treatment of the above conditions, with preferred levels of about 0.1 mg to about 1,000 mg. The amount of active ingredient that may be combined with the carrier materials to produce a single dosage form will vary depending upon the host treated and the particular mode of administration. Typically, in vitro dosage-effect results provide useful guidance on the proper doses for patient administration. Studies in animal models are also helpful. The considerations for determining the proper dose levels are well known in the art.

It is understood; however, that a specific dose level for any particular patient will depend upon a variety of factors including the activity of the specific compound employed, the age, body weight, general health, sex, diet, time of administration, rate of excretion, drug combination, and the severity of the particular disease being treated and form of administration.

To effectively treat alopecia or promote hair growth, the compounds and pharmaceutical compositions must readily affect the targeted areas. For these purposes, the compounds are preferably administered topically to the skin.

For topical application to the skin, the compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water. Alternatively, the compounds can be formulated into suitable lotions or creams containing the active compound suspended or dissolved in, for example, a mixture of one or more of the following: mineral oil, sorbitan monostearate, polysorbate 60, cetyl ester wax, cetearyl alcohol, 2-octyldodecanol, benzyl alcohol and water.

The compounds can be administered with other hair revitalizing agents. Specific dose levels for the other hair revitalizing agents will depend upon the factors previously stated and the effectiveness of the drug combination. Other routes of administration known in the pharmaceutical art are also contemplated by this invention.

The present invention contemplates employing the compounds of the present invention for use in compositions and methods to prevent and/or treat a neurological disorder in an animal, and for use in compositions and methods to treat alopecia and promote hair growth in an animal, and all other uses suggested in this specification.

The compounds of the present invention can be used for the treatment of a disease such as peripheral neuropathy caused by physical injury or disease state, physical damage to the brain, physical damage to the spinal cord, stroke associated with brain damage, Alzheimer's Disease, Parkinson's Disease, and amyotrophic lateral sclerosis.
The compounds can also be used for treating the above-mentioned neuropathies, neurological disorders, and neurological damage.

They may also be used for treating alopecia or promoting hair growth in an animal, which comprises administering to said animal an effective amount of an N-heterocyclic carboxylic acid or carboxylic acid isostere.

The compounds are particularly useful for treating male pattern alopecia, alopecia senilis, alopecia areata, alopecia resulting from skin lesions or tumors, alopecia resulting from cancer therapy such as chemotherapy and radiation, and alopecia resulting from systematic disorders such as nutritional disorders and internal secretion disorders.

It is understood, however, that a specific dose level for any particular patient will depend upon a variety of factors including the activity of the specific compound employed, the age, body weight, general health, sex, diet, time of administration, rate of excretion, drug combination, and the severity of the particular disease or disorder being treated and form of administration.

### MPTP Model of Parkinson's Disease in Mice

MPTP lesioning of dopaminergic neurons in mice was used as an animal model of Parkinson's Disease. Four week old male CD1 white mice were dosed i.p. with 30 mg/kg of MPTP for 5 days. The inventive compounds (4 mg/kg), or vehicle, were administered s.c. along with the MPTP for 5 days, as well as for an additional 5 days following cessation of MPTP treatment. At 18 days following MPTP treatment, the animals were sacrificed and the striata were dissected and homogenized. Immunostaining was performed on saggital and coronal brain sections using anti-tyrosine hydoxylase Ig to quantitate survival and recovery of dopaminergic neurons. In animals treated with MPTP and vehicle, a substantial loss of functional dopaminergic terminals was observed as compared to non-lesioned animals. In another protocol, test compounds were administered only subsequent to MPTP-induced lesioning. Thus, after animals were treated with MPTP for 5 days, an additional 3 days passed before beginning oral drug treatment on day 8. Animals were treated with the inventive compounds (0.4 mg/kg), administered orally, once a day for 5 days total. On day 18, the animals were sacrificed and analyzed as described above.

Table V presents the percent recovery of dopaminergic neurons in the first (concurrent dosing) paradigm in animals receiving carboxylic acid or carboxylic acid isostere compounds of the present invention.

Table V, below, shows the remarkable neuroregenerative effects of the inventive carboxylic acid or carboxylic acid isostere related compounds illustrating the neurotrophic capability of carboxylic acid isosteres as a class showing that lesioned animals receiving the carboxylic acid or carboxylic acid isostere compounds provide a remarkable recovery of TH-stained dopaminergic neurons.

**Table V - BPTP Neurodegenerative Model**

| | % Recovery |
|---|---|
| Compound A | 26.7 % |
| Compound B | ND |
| Compound C | 24.4 % |
| Compound D | 23.2 % |
| Compound E | 19.6 % |
| Compound F | 34.1 % |
| Compound G | 46.5 % |
| Compound H | 14.0 % |
| Compound I | ND |

Percent striatal innervation density was quantitated in brain sections with an anti-tyrosine hydroxylase immunoglobulin, which is indicative of functional dopaminergic neurons. The striatal innervation density of 23% for animals pretreated with only a vehicle and administered a vehicle orally during treatment, is indicative of normal non-lesioned striatal tissue. Striatal innervation density is reduced to 5% for animals pretreated with MPTP and administered a vehicle orally during treatment, and is indicative of MPTP-induced lesioning. Surprisingly, striatal innervation density is increased 8-13% for animals pretreated with MPTP and administered 0.4 mg/kg of an inventive compound orally during treatment, indicating substantial neuronal regeneration after induction of MPTP-derived lesions.

The following examples are illustrative of preferred embodiments of the invention and are not to be construed as limiting the invention thereto. All polymer molecular weights are mean average molecular weights. All percentages are based on the percent by weight of the final delivery system or formulation prepared unless otherwise indicated and all totals equal 100% by weight.

### EXAMPLES

The inventive compounds may be prepared by a variety of synthetic sequences that utilize established chemical transformations. A general pathway to the present compounds is described in Scheme I. N-glyoxylproline derivatives may be prepared by reacting L-proline methyl ester with methyl oxalyl chloride as shown in Scheme I. The resulting oxamates may be reacted with a variety of carbon nucleophiles to obtain compounds used in the present invention.

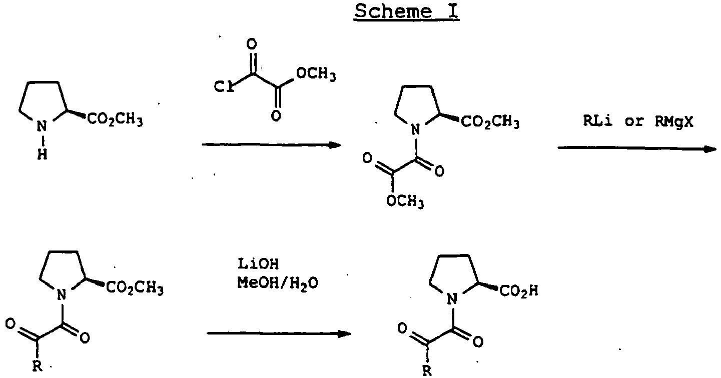

### EXAMPLE 1 (Compound 137)

Synthesis of (2S)-1-(3,3-dimethyl-1,2-dioxopentyl)-2-pyrrolidinecarboxylate.

### a. Synthesis of (2S)-1-(1,2-dioxo-2-methoxyethyl)-2-pyrrolidinecarboxylate.

A solution of L-proline methyl ester hydrochloride (3.08 g; 18.60 mmol) in dry methylene chloride was cooled to 0°C and treated with triethylamine (3.92 g; 38.74 mmol; 2.1 eq). After stirring the formed slurry under a nitrogen atmosphere for 15 min, a solution of methyl oxalyl chloride (3.20 g; 26.12 mmol) in methylene chloride (45 mL) was added dropwise. The resulting mixture was stirred at 0°C for 1.5 hr. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of cis-trans amide rotamers; data for trans rotamer given. ¹H NMR (CDCl₃): δ 1.93 (dm, 2H); 2.17 (m, 2H); 3.62 (m, 2H); 3.71 (s, 3H); 3.79, 3.84 (s, 3H total); 4.86 (dd, 1H, J = 8.4, 3.3).

### b. Synthesis of methyl (2S)-1-(1,2-dioxo-3,3-dimethylpentyl)-2-pyrrolidinecarboxylate.

A solution of methyl (2S)-1-(1,2-dioxo-2-methoxyethyl)-2-pyrrolidinecarboxylate (2.35 g; 10.90 mmol) in 30 mL of tetrahydrofuran (THF). was cooled to - 78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): δ 0.88 (t, 3H); 1.22, 1.26 (s, 3H each); 1.75 (dm, 2H); 1.87-2.10 (m, 3H); 2.23 (m, 1H); 3.54 (m, 2H); 3.76 (s, 3H); 4.52 (dm, 1H, J = 8.4, 3.4).

### c. Synthesis of (2S)-1-(1,2-dioxo-3,3-dimethylpentyl)-2-pyrrolidinecarboxylic acid (Compound 137).

A mixture of methyl (2S)-1-(1,2-dioxo-3,3-dimethylpentyl)-2-pyrrolidinecarboxylate (2.10 g; 8.23 mmol), 1 N LiOH (15 mL), and methanol (50 mL) was stirred at 0°C for 30 min and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃): δ 0.87 (t, 3H); 1.22, 1.25 (s, 3H each); 1.77 (dm, 2H); 2.02 (m, 2H); 2.17 (m, 1H) ; 2.25 (m, 1H) ; 3.53 (dd, 2H, J = 10. 4, 7.3); 4.55 (dd, 1H, J = 8.6, 4.1).

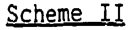

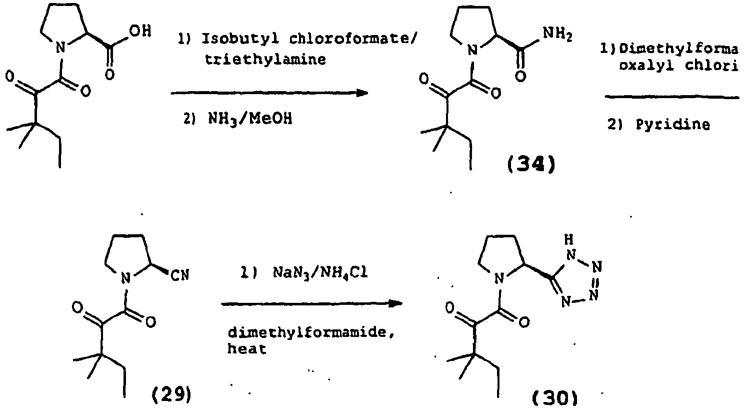

Inventive compounds containing bridged rings may be synthesized using the above synthetic schemes by substituting the substrates containing the N-heterocyclic ring structures with comparable substrates containing bridged ring structures.

### Example 2

### in Vivo Hair Generation Test Witch C57 Black 6 Mice

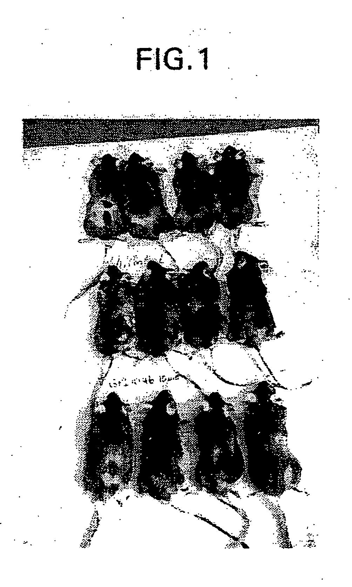

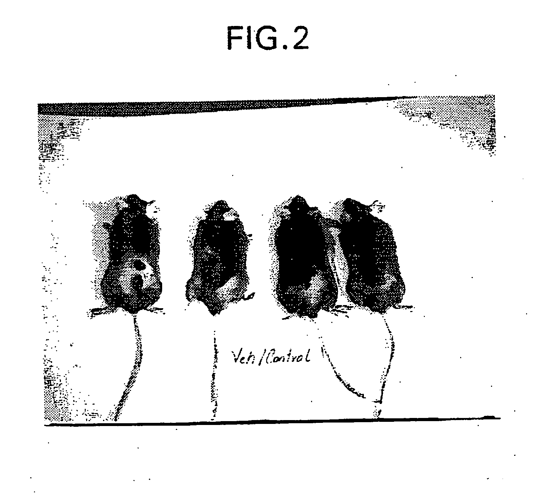

C57 black 6 mice were used to demonstrate the hair revitalizing properties of the N-heterocyclic carboxylic acids or carboxylic acid isosteres. Referring now to FIGS. 1 and 2 of the drawings, C57 black 6 mice, approximately 7 weeks old, had an area of about 2 inches by 2 inches on their hindquarters shaved to remove all existing hair. Care was taken not to nick or cause abrasion to the underlaying dermal layers. The animals were in anagen growth phase, as indicated by the pinkish color of the skin. Referring now to FIG. 2, four animals per group were treated by topical administration with 20% propylene glycol vehicle (FIG. 2), or neuroimmunophilin FKBP ligands dissolved in the vehicle. The animals were treated with vehicle or neuroimmunophilin ligands every 48 hours (3 applications total over the course of 5 days) and the hair growth was allowed to proceed for 6 weeks. Hair growth was quantitated by the percent of shaved area covered by new hair growth during this time period.

FIG. 2 shows that animals treated with vehicle exhibited only a small amount of hair growth in patches or tufts, with less than 3% of the shaved area covered with new growth.

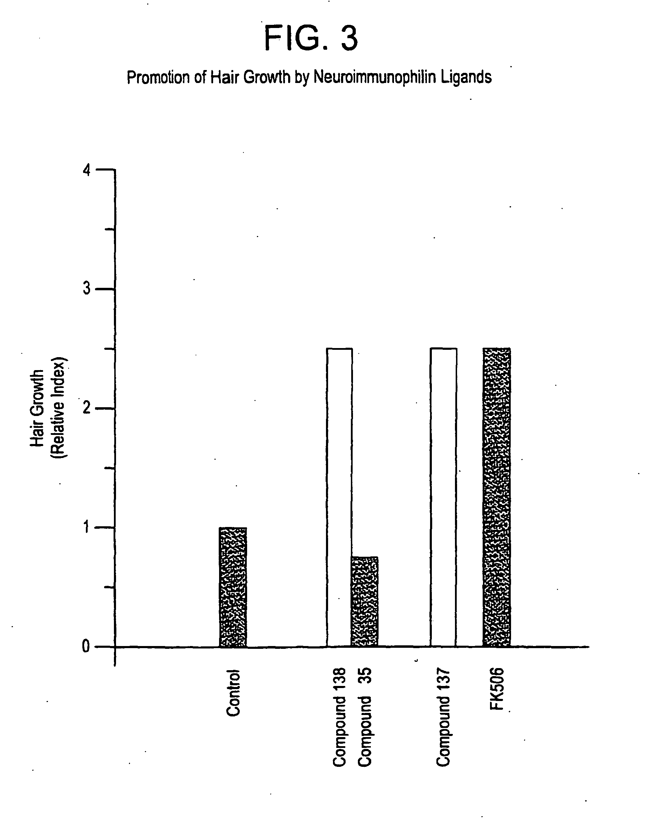

In contrast, FIG. 3 shows that animals treated for 2 weeks with the N-heterocyclic carboxylic acid compounds i.e. compound A (137), compound B (138), and compound G (35) exhibited dramatic hair growth, covering greater than 25% of the shaved area in all animals for two of the compounds.

FIG. 3 shows the relative hair growth on shaven C57 black 6 mice 14 days after being treated with one of three N-heterocyclic carboxylic acids or carboxylic acid isosteres. The mice had a 2 x 2 inch region on their backside shaved to remove all hair. Care was taken not to nick or cause abrasion to the underlying dermal layers. Compounds at a concentration of 1 µmole per milliliter were carefully applied to the shaved area of the mice (5 mice per group) three times per week. Hair growth was evaluated 14 days after initiation of drug treatment. The relative scale for assessing hair growth is as follows:
0 = no growth;
1 = beginning of growth in small tufts;
2 = hair growth covering over <25% of shaved area;
3 = hair growth covering over >25% of shaved area, but less than 50% of shaved area;
4 = hair growth covering over >50% of shaved area, but less than 75% of shaved area;
5 = complete hair growth of shaved area.

### Example 3

A lotion comprising the following composition may be prepared.

| | (%) |
|---|---|
| 95% Ethanol | 80.0 |
| an N-heterocyclic carboxylic acid or carboxylic acid isostere | 10.0 |
| α-Tocopherol acetate | 0.01 |
| Ethylene oxide (40 mole) adducts of hardened castor oil | 0.5 |
| purified water | 9.0 |
| perfume and dye | q.s. |

Into 95% ethanol are added an N-heterocyclic carboxylic acid or carboxylic acid isostere, α-tocopherol acetate, ethylene oxide (40 mole) adducts of hardened castor oil, perfume and a dye. The resulting mixture is stirred and dissolved, and purified water is added to the mixture to obtain a transparent liquid lotion.

5 mL of the lotion may be applied once or twice per day to a site having marked baldness or alopecia.

### Example 4

A lotion comprising the following composition shown may be prepared.

| | (%) |
|---|---|
| 95% Ethanol | 80.0 |
| an N-heterocyclic carboxylic acid or carboxylic acid isostere | 0.005 |
| Hinokitol | 0.01 |
| Ethylene oxide (40 mole) adducts of hardened castor oil | 0.5 |
| Purified water | 19.0 |
| Perfume and dye | q.s. |

Into 95% ethanol are added an N-heterocyclic carboxylic acid or carboxylic acid isostere, hinokitol, ethylene oxide (40 mole) adducts of hardened castor oil, perfume, and a dye. The resulting mixture is stirred, and purified water is added to the mixture to obtain a transparent liquid lotion.

The lotion may be applied by spraying once to 4 times per day to a site having marked baldness or alopecia.

### Example 5

An emulsion may be prepared from A phase and B phase having the following compositions.

| **(A phase)** | (%) |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitan monooleate | 1.0 |
| an N-heterocyclic carboxylic acid or carboxylic acid isostere | 0.01 |

| **(B phase)** | (%) |
|---|---|
| Glycerine | 10.0 |
| Purified water | 69.0 |
| Perfume, dye, and preservative | q.s. |

The A phase and the B phase are respectively heated and melted and maintained at 80°C. Both phases are then mixed and cooled under stirring to normal temperature to obtain an emulsion.

The emulsion may be applied by spraying once to four times per day to a site having marked baldness or alopecia.

### Examgle 6

A cream may be prepared from A phase and B phase having the following compositions.

| **(A Phase)** | (%) |
|---|---|
| Fluid paraffin | 5.0 |
| Cetostearyl alcohol | 5.5 |
| Petrolatum | 5.5 |
| Glycerine monostearate | 33.0 |
| Polyoxyethylene (20 mole) 2-octyldodecyl ether | 3.0 |
| Propylparaben 0.3 | 0.3 |

| **(B Phase)** | (%) |
|---|---|
| an N-heterocyclic carboxylic acid or carboxylic acid isostere | 0.8 |
| Glycerine | 7.0 |
| Dipropylene glycol | 20.0 |
| Polyethylene glycol 4000 | 5.0 |
| Sodium Hexametaphosphate | 0.005 |
| Purified water | 44.895 |

The A phase is heated and melted, and maintained at 70°C. The B phase is added into the A phase and the mixture is stirred to obtain an emulsion. The emulsion is then cooled to obtain a cream.

The cream may be applied once to 4 times per day to a site having marked baldness or alopecia.

### Example 7

A liquid comprising the following composition may be prepared.

| | (%) |
|---|---|
| Polyoxyethylene butyl ether | 20.0 |
| Ethanol | 50.0 |
| an N-heterocyclic carboxylic acid or carboxylic acid isostere | 0.001 |
| Propylene glycol | 5.0 |
| Polyoxyethylene hardened castor oil derivative (ethylene oxide 80 mole adducts) | 0.4 |
| Perfume | q.s. |
| Purified water | q. s. |

Into ethanol are added polyoxypropylene butyl ether, propylene glycol, polyoxyethylene hardened castor oil, an N-heterocyclic carboxylic acid or carboxylic acid isostere, and perfume. The resulting mixture is stirred, and purified water is added to the mixture to obtain a liquid.

The liquid may be applied once to 4 times per day to a site having marked baldness or alopecia.

### Example 8

A shampoo comprising the following composition may be prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| an N-heterocyclic carboxylic acid or carboxylic acid isostere | 5.0 |
| Ethanol | 2.0 |
| Perfume | 0.3 |
| Purified water | 69.7 |

Into 69.7 of purified water are added 5.0 g of sodium laurylsulfate, 5.0 g of triethanolamine laurylsulfate, 6.0 g of betaine lauryldimethyl-aminoacetate. Then a mixture obtained by adding 5.0 g of an N-heterocyclic carboxylic acid or carboxylic acid isostere, 5.0 g of polyethylene glycol, and 2.0 g of ethylene glycol distearate to 2.0 g of ethanol, followed by stirring, and 0.3 g of perfume are successively added. The resulting mixture is heated and subsequently cooled to obtain a shampoo.

The shampoo may be used on the scalp once or twice per day.

### Example 9

A patient is suffering from alopecia senilis.

A compound of the present invention a pharmaceutical composition comprising the same, may be administered to the patient. Increased hair growth is expected to occur following treatment.

### Example 10

A patient is suffering from male pattern alopecia.

A compound of the present invention or a pharmaceutical composition comprising the same may be administered to the patient. Increased hair growth is expected to occur following treatment.

### Example 11

A patient is suffering from alopecia areata.

A compound of the present invention or a pharmaceutical composition comprising the same, may be administered to the patient. Increased hair growth is expected to occur following treatment.

### Example 12

A patient is suffering from hair loss caused by skin lesions. A compound of the present invention or a pharmaceutical composition comprising the same, may be administered to the patient. Increased hair growth is expected to occur following treatment.

### Example 13

A patient is suffering from hair loss caused by tumors. A compound of the present invention or a pharmaceutical composition comprising the same, may be administered to the patient. Increased hair growth is expected to occur following treatment.

### Example 14

A patient is suffering from hair loss caused by a systematic disorder, such as a nutritional disorder or an internal secretion disorder.

A compound of the present invention or a pharmaceutical composition comprising the same, may be administered to the patient. Increased hair growth is expected to occur following treatment.

### Example 15

A patient is suffering from hair loss caused by chemotherapy. A compound of the present invention or a pharmaceutical composition comprising the same, may be administered to the patient. Increased hair growth is expected to occur following treatment.

### Example 16

A patient is suffering from hair loss caused by radiation. A compound of the present invention or a pharmaceutical composition comprising the same may, be administered to the patient. Increased hair growth is expected to occur following treatment.

### Example 17

A patient is suffering from a neurodegenerative disease. A compound of the present invention or a pharmaceutical composition comprising the same is administered. It would be expected that the patient would improve their condition or recover.

### Example 18

A patient is suffering from a neurological disorder. A compound of the present invention or pharmaceutical compositions comprising same is administered. It would be expected that the patient would improve their condition or recover.

### Example 19

A patient is suffering from stroke.

A compound of the present invention or pharmaceutical compositions comprising same is administered. It would be expected that the patient would improve their condition or recover.

### Example 20

A patient is suffering from Parkinson's Disease.

A compound of the present invention or pharmaceutical compositions comprising same is administered. It would be expected that the patient would improve their condition or recover.

### Example 21

A patient is suffering from Alzheimer's Disease.

A compound of the present invention or pharmaceutical compositions comprising same is administered. It would be expected that the patient would improve their condition or recover.

### Example 22

A patient is suffering from a peripheral neuropathy. A compound of the present invention or pharmaceutical compositions comprising same is administered. It would be expected that the patient would improve their condition or recover.

### Example 23

A patient is suffering from amyotrophic lateral sclerosis. A compound of the present invention or pharmaceutical compositions comprising same is administered. It would be expected that the patient would improve their condition or recover.

### Example 24

A patient is suffering from a spinal injury. A compound of the present invention or pharmaceutical compositions comprising same is administered. It would be expected that the patient would improve their condition or recover.

### Example 25

A patient is at risk of suffering from a neurodegenerative disease or neurological disorder. A compound of the present invention or a pharmaceutical composition comprising the same is propnelactically administered. It would be expected that the patient would be prevented from some or all of the effects of the disease or disorder, or would significally improve their condition or recover over patients who were not pre-treated.

The invention being thus described, it will be obvious that the same may be varied in many ways. Such variations are not to be regarded as a departure from the scope of the invention and all such modification are intended to be included within the scope of the following claims.

## Claims

1. A pharmaceutical composition, comprising
a) an effective amount of a compound according to formula 3, 5, 8, 21, or 137: 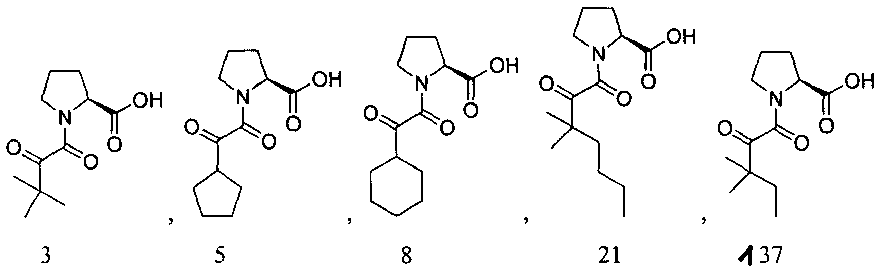 or a pharmaceutically acceptable salt thereof, and
b) a pharmaceutically acceptable carrier.

2. The pharmaceutical composition according to claim 1 wherein the compound is formula 137

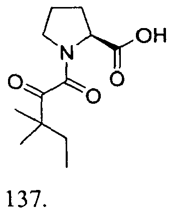

3. The pharmaceutical composition according to any of claims 1-3, further comprising an additional neurotrophic factor.

4. The pharmaceutical composition according to claim 3, wherein said additional neurotrophic factor is selected from neurotrophic growth factor, brain derived growth factor, glial derived growth factor, cilial neurotrophic factor, insulin growth factor and active truncated derivatives thereof, acidic fibroblast growth factor, basic fibroblast growth factor, platelet-derived growth factors, neurotropin-3, and neurotropin 4/5.

5. The compound as defined in Claim 1-2 or a pharmaceutically acceptable salt thereof for use in a method for treating a neurological disorder in an animal, for stimulating growth of damaged peripheral nerves, for promoting neuronal regeneration, and/or for preventing neurodegeneration in the animal.

6. The compound for use of claim 5, wherein the neurological disorder is selected from peripheral neuropathies caused by physical injury or disease state, physical damage to the brain, physical damage to the spinal cord, stroke associated with brain damage, and neurological disorders relating to neurodegeneration.

7. The compound for use of claim 5, wherein the neurological disorder is selected from Alzheimer's Disease, Parkinson's Disease, amyotrophic lateral sclerosis, trigeminal neuralgia, glossopharyngeal, neuralgia, Bell's Palsy, myasthenia gravis, muscular dystrophy, progressive muscular atrophy, progressive bulbar inherited muscular atrophy, herniated, ruptured, or prolapsed invertebrate disk syndromes, cervical spondylosis, plexus disorders, thoracic outlet destruction syndromes, Gullain-Barré syndrome, and sciatic nerve lesions.

8. A compound as defined in any of claims 1-3 or a pharmaceutically acceptable salt thereof for use in a method for treating alopecia and/or promoting hair growth in an animal.

9. The pharmaceutical composition according to claim 2 wherein the composition is formulated for topical administration.

10. The pharmaceutical composition according to claim 10 wherein the topical application is to the skin.

11. The pharmaceutical composition according to claim 10 wherein the pharmaceutical formulation is chosen from an ointment, a lotion, a cream, an emulsion, a liquid, or a shampoo.

12. The pharmaceutical composition according to claim 11 wherein emulsion is delivered by spray.

## Patentansprüche

1. Pharmazeutische Zusammensetzung, umfassend
a) eine wirksame Menge einer Verbindung nach Formel 3, 5, 8, 21 oder 137: 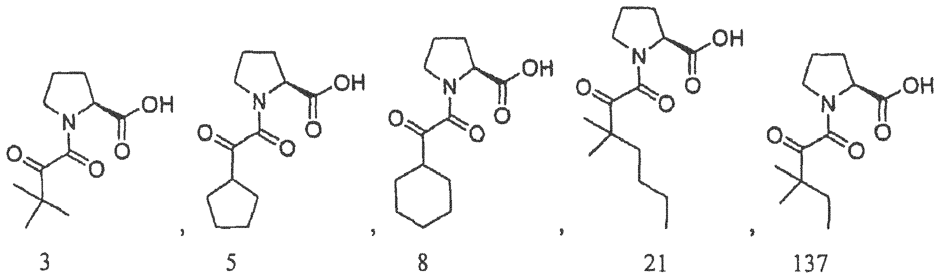 oder ein pharmazeutisch verträgliches Salz hiervon und
b) einen pharmazeutisch verträglichen Träger.

2. Pharmazeutische Zusammensetzung nach Anspruch 1, wobei die Verbindung die 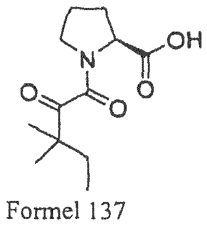 ist 137.

3. Pharmazeutische Zusammensetzung nach Anspruch 1 oder 2, weiterhin umfassend einen zusätzlichen neurotrophen Faktor.

4. Pharmazeutische Zusammensetzung nach Anspruch 3, wobei der zusätzliche neurotrophe Faktor ausgewählt ist aus neurotrophen Wachstumsfaktor, vom Gehirn stammendem Wachstumsfaktor, von Gliazellen stammendem Wachstumsfaktor, ziliärneurotrophen Faktor, Insulin-Wachstumsfaktor und aktiven, trunkierten Derivaten hiervon, sauren Fibroblasten-Wachstumsfaktor, basischen Fibroblasten-Wachstumsfaktor, von Blutplättchen stammenden Wachstumsfaktoren, Neurotropin-3 und Neurotropin 4/5.

5. Verbindung nach Anspruch 1-2 oder ein pharmazeutisch verträgliches Salz hiervon zur Verwendung in einem Verfahren zur Behandlung einer neurologischen Störung bei einem Tier, zur Stimulierung des Wachstums von beschädigten peripheren Nerven, zur Förderung neuronaler Regeneration und/oder zur Verhinderung von Neurodegeneration in dem Tier.

6. Verbindung zur Verwendung nach Anspruch 5, wobei die neurologische Störung ausgewählt ist aus, durch Verletzungen oder einen Krankheitszustand verursachten peripheren Neuropathien, einer körperlichen Schädigung des Gehirns, einer körperlichen Schädigung des Rückenmarks, einem mit Gehirnschaden verbundenen Schlaganfall, und im Zusammenhang mit Neurodegeneration stehenden neurologischen Störungen.

7. Verbindung zur Verwendung nach Anspruch 5, wobei die neurologische Störung ausgewählt ist aus Alzheimer-Krankheit, Parkinson-Krankheit, amyotropher Lateralsklerose, Trigeminusneuralgie, Glossopharyngeusneuralgie, Bell-Lähmung, Myasthenia gravis, Muskeldystrophie, progressiver Muskelatrophie, vererbter, progressiven bulbären Muskelatrophie, Syndromen betreffend gebrochene, gerissene oder prolabierte Bandscheiben, zervikaler Spondylose, Störungen des Nervenplexus, Thoracic-outlet-Zerstörungssyndromen, Gullain-Barré-Syndrom und Ischiasnervenläsionen.

8. Verbindung nach einem der Ansprüche 1-3 oder ein pharmazeutisch verträgliches Salz hiervon zur Verwendung in einem Verfahren zur Behandlung von Alopezie und/oder zur Förderung von Haarwachstum in einem Tier.

9. Pharmazeutische Zusammensetzung nach Anspruch 2, wobei die Zusammensetzung zur topischen Anwendung formuliert ist.

10. Pharmazeutische Zusammensetzung nach Anspruch 10, wobei die topische Applikation auf der Haut erfolgt.

11. Pharmazeutische Zusammensetzung nach Anspruch 10, wobei die pharmazeutische Formulierung ausgewählt ist aus einer Salbe, einer Lotion, einer Creme, einer Emulsion, einer Flüssigkeit oder einem Shampoo.

12. Pharmazeutische Zusammensetzung nach Anspruch 11, wobei die Emulsion mittels Spray verabreicht wird.

## Revendications

1. Composition pharmaceutique comprenant
a) une quantité efficace d'un composé selon la formule 3, 5, 8, 21 ou 137 : 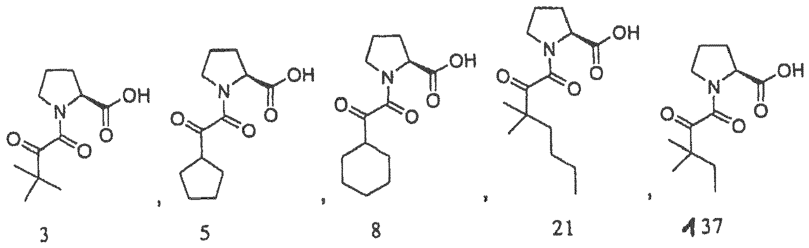 ou un sel pharmaceutiquement acceptable de celui-ci, et
b) un véhicule pharmaceutiquement acceptable.

2. Composition pharmaceutique selon la revendication 1, dans laquelle le composé répond à la formule 137

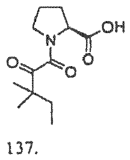

3. Composition pharmaceutique selon l'une quelconque des revendications 1 à 3, comprenant en outre un facteur neurotrophique supplémentaire,

4. Composition pharmaceutique selon la revendication 3, dans laquelle ledit facteur neurotrophique supplémentaire est choisi parmi le facteur de croissance neurotrophique, le facteur de croissance dérivé du cerveau, le facteur de croissance dérivé de la glie, le facteur neurotrophique ciliaire, le facteur neurotrophique de l'insuline et des dérivés actifs tronqués de celle-ci, le facteur de croissance des fibroblastes acide, le facteur de croissance des fibroblastes basique, les facteurs de croissance dérivés de plaquettes, la neurotropine-3 et la neurotropine 4/5.

5. Composé tel que défini dans la revendication 1-2, ou sel pharmaceutiquement acceptable de celui-ci, pour son utilisation dans une méthode pour le traitement d'un trouble neurologique chez un animal, pour stimuler la croissance de nerfs périphériques endommagés, pour promouvoir la régénération neuronale et/ou pour empêcher une neurodégénérescence chez l'animal.

6. Composé pour son utilisation selon la revendication 5, dans lequel le trouble neurologique est choisi pa les neuropathies périphériques par un traumatisme physique ou un état maladif, une lésion physique du cerveau, une lésion physique de la moelle épinière, un accident vasculaire cérébral associé à une lésion cérébrale et des troubles neurologiques apparentés à une neurodégénérescence.

7. Composé pour son utilisation selon la revendication 5, dans lequel le trouble neurologique est choisi parmi la maladie d'Alzheimer, la maladie de Parkinson, la sclérose latérale amyotrophique, la névralgie trigéminale, le glossopharyngien, la névralgie, la paralysie de Bell, la myasthénie, la dystrophie musculaire, l'atrophie musculaire progressive, l'atrophie musculaire bulbaire progressive héréditaire, les syndromes de l'herniation, de la rupture ou du prolapsus de disque intervertébral, la spondylose cervicale, les troubles du plexus, les syndromes de destruction de la traversée thoracobrachiale, le syndrome de Guillain-Barré et les lésions du nerf sciatique.

8. Composé tel que défini dans l'une quelconque des revendications 1-3 ou un sel pharmaceutiquement acceptable de celui-ci, pour son utilisation dans une méthode pour traiter la calvitie et/ou promouvoir la croissance de cheveux chez un animal.

9. Composition pharmaceutique selon la revendication 2, dans laquelle la composition est formulée pour une administration topique.

10. Composition pharmaceutique selon la revendication 10, dans laquelle l'application topique est réalisée sur la peau.

11. Composition pharmaceutique selon la revendication 10, dans laquelle la forme pharmaceutique est choisie parmi une pommade, une lotion, une crème, une émulsion, un liquide ou un shampoing.

12. Composition pharmaceutique selon la revendication 11, dans laquelle l'émulsion est délivrée par pulvérisation.
